# EUROPEAN PATENT SPECIFICATION

(11) **EP 3 047 912 B1**
(45) Date of publication and mention of the grant of the patent: **13.12.2017**
(21) Application number: 15152564.9
(22) Date of filing: 26.01.2015
(51) Int. Cl.: B05B 12/00

(54) **Device and method for controlling a spray pattern**
Vorrichtung und Verfahren zum Überwachen eines Sprühstrahls
Dispositif et procédé pour contrôler un jet de liquide

(43) Date of publication of application: 27.07.2016
(73) Proprietor: Inman S.r.l., 20122 Milano (IT)
(72) Inventor: Bettinelli, Vincenzo, I-20122 MILANO (MI) (IT)
(74) Representative: Marietti, Andrea

(56) References cited:
- WO-A1-2006/007706
- WO-A1-2011/063808
- DE-A1- 10 138 167
- US-A- 5 047 612

## Description

The present invention relates to the check of fluid nebulizing dispensers (or actuators) mainly dedicated to pharmaceutical, medical or cosmetic sectors.

The dispensing quality of a pharmaceutical or cosmetic product dispensed from these devices is a key requirement of the market.

Not only do the proper nebulization and the correct amount of dispensed product determine this quality, but also the dispensing shape/dimension/direction of this product that should preferably take the shape of a cone downstream of the dispenser. The known art provides a manual sampled check of some dispensing devices by inserting a colored liquid for simulating the product inside the dispenser and dispensing such liquid against a detecting absorbent surface.

An operator examines and evaluates the spot of colored liquid in order to determine the quality of the dispenser under test.

Clearly, this is a time-consuming check which requires labor and causes the waste of the randomly chosen containers, thereby preventing them from being put on the market. Moreover, this kind of check does not allow to test all the manufactured dispensers. US 5047612 discloses an apparatus for controlling the powder deposition and deposit pattern in a plasma spray process.

It is an object of the present invention to provide a system for recognizing the proper shape of the dispensing cone of a nebulizing, or spraying, dispenser, or the like. In particular, it is an object of the present invention to provide a system able to perform a non-invasive check thereby preventing the contamination of the dispenser itself, such that the latter can be put on the market. Such objects are achieved by a system and a related operating method according to the appended claims.

In particular, according to an aspect of the present invention, a device for checking the distribution of fluid ejected from a fluid nebulizing dispenser, of the type comprising a discharging nozzle of nebulized fluid, comprises means for detecting the temperature difference between at least part of the nebulized fluid ejected from the dispenser and at least one contrast space, and/or the temperature difference caused to the contrast space by the nebulized fluid ejected from the dispenser. In this case, the contrast space is arranged so as to be at least partially incident to the nebulized fluid.

In other words, some fluid is nebulized from a discharging nozzle and placed in contact with a contrast space. Appropriate means detect the temperature difference between the fluid and the contrast space, thus identifying the fluid path downstream of the discharging nozzle.

According to an aspect of the present invention, the contrast space comprises a contact surface having a temperature different from said nebulized fluid ejected from said dispenser.

Therefore, the nebulized fluid can be dispensed against said contact surface such that the above mentioned detecting means can identify the mark left on the surface by the nebulized fluid. In particular, the detecting means identify such a mark because of its temperature which is different from the rest of the contact surface not touched by the nebulized fluid.

According to an aspect of the present invention, the contact surface is thermally connected to heating and/or cooling means.

Such heating/cooling means allow to lead the contact surface to a temperature different from the nebulized fluid.

According to another aspect of the invention, the contrast space comprises a volumetric portion of the environment downstream of the discharging nozzle.

In other words, the temperature difference between the fluid and the surrounding space allows the detecting means to identify the presence of the fluid nebulized by the dispenser downstream of the discharging nozzle.

According to an aspect of the present invention, the detecting means comprise at least one thermographic sensor to capture at least one image of the temperature difference between at least part of said nebulized fluid ejected from said dispenser and said contrast space and/or the projection of said image onto said contrast space. The thermographic sensors, typically thermographic cameras, make the capture of an image of the nebulized fluid possible.

Further, or alternatively, the detecting means can comprise temperature sensors.

According to an aspect of the present invention, the contact surface is at least partially covered by a thermal-insulating material.

This allows to minimize the waiting times between the check of two different dispensers. This waiting time depends on the time taken to lead the contact surface to a uniform temperature in response to a check, that is the time taken to erase the mark of the fluid nebulized by a dispenser.

Preferably, such thermal-insulating material is film-like. According to a further aspect this thermal-insulating material is Teflon.

Another aspect of the present invention relates to a method for checking the distribution of the fluid ejected from a fluid nebulizing container according to one or more of the above aspects, comprising the steps of:
a) ejecting a nebulized fluid from said discharging nozzle;
b) detecting the temperature difference between at least part of the nebulized fluid and the contrast space and/or the temperature variation caused to the contrast space by the nebulized fluid.

Hereinafter, referring to the appended figures, exemplary and non-limiting embodiments of the present invention will be described, wherein:
- Figure 1 is a perspective view of an embodiment of the present invention;
- Figure 2 is a schematic view of a second embodiment of the present invention;
- Figure 3 is a perspective view of a modification of the embodiment of figure 2.

Referring to figures, a device 1 according to the present invention allows to check the distribution 2 of a fluid 3b ejected from a dispenser 4.

The dispenser 4 is known in the art and comprises a body 4a adapted to contain a fluid 3a, and a nozzle 4b adapted to nebulize and discharge such fluid 3b from the dispenser 4.

It can be clearly seen that the dispenser 4 shown in figures is only for illustration since the present invention is able to check differently-shaped dispensers and, in particular, a general dispenser provided with a discharging nozzle of a nebulized fluid.

Typically, during the normal use of the dispenser 4, the fluid 3a, 3b is selected from a cosmetic (e.g. a perfume) or a pharmaceutical or medical fluid. Hereinafter, for the sake of convenience, the same fluid 3a, 3b is referred to by means of two different reference numerals. In particular, when the fluid is contained inside the dispenser 4 (or in any case before being dispensed from the nozzle 4b), it is indicated as fluid 3a whereas, once it has been ejected from the dispenser 4 through the nozzle 4b, it is indicated as fluid 3b.

According to an aspect of the present invention, to carry out the quality control of the dispenser 4, the fluid 3a, 3b preferably is an inert gas (for example suitably filtered air). Since the inert gas does not contaminate the dispensers 4, the same dispensers 4 undergoing a check may subsequently be put on the market.

The fluid 3b can be dispensed from the discharging nozzle 4b according to different methods. For example, according to an embodiment, the single discharging nozzle 4b, fluidically connected to a fluid source 3a, is tested. Adjusting means 6b can be provided in order to adjust the dispensing pressure of the fluid 3b.

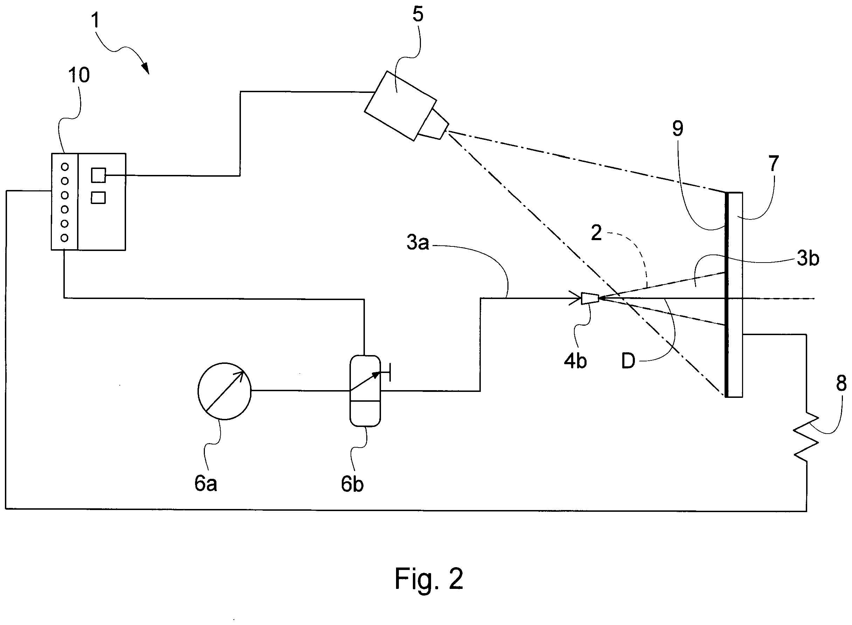

In particular, a compressor 6a and a valve 6b respectively acting as a fluid source 3a and adjusting means, are shown in figure 2. Alternatively, the compressor 6a can be adjustable so as to change the dispensing pressure in order to act as both source and adjusting means, simultaneously.

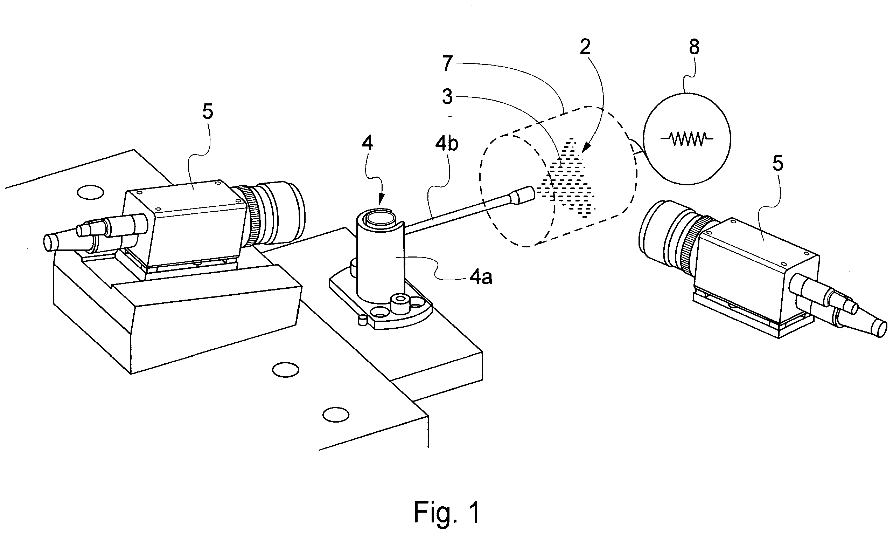

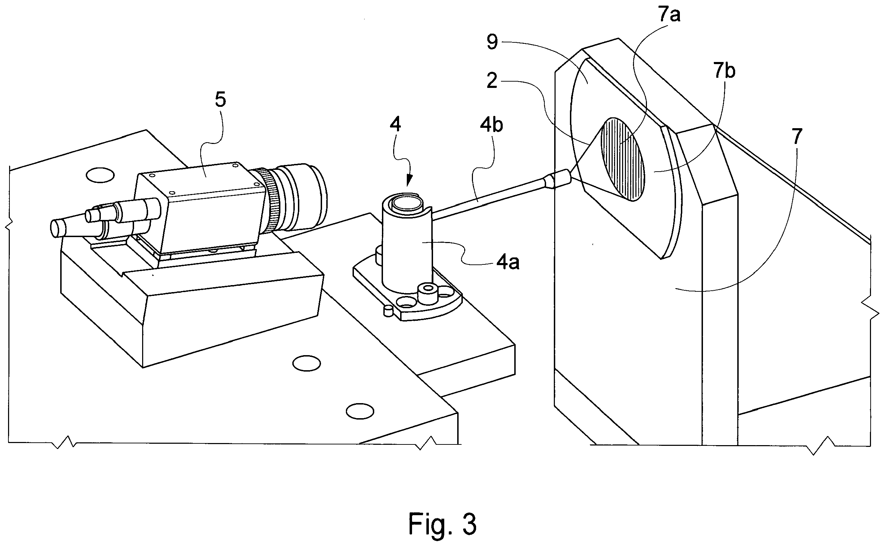

In the embodiments shown in figures 1 and 3, the discharging nozzle is mounted instead on the body 4a of the dispenser 4. The dispensing of nebulized fluid 3b is controlled by operators, or suitable automated actuators, not shown, by acting on the body 4a. Alternatively, in place of the dispenser body 4a, the nozzle 4b can be mounted on a specific element adapted to contain the fluid and to couple with the nozzle itself.

The device 1 according to the present invention further comprises detecting means 5 for detecting the temperature difference between at least part of the nebulized fluid 3b ejected from the dispenser 4 and at least one contrast space 7, and/or the temperature difference which is caused by the nebulized fluid 3b ejected from the dispenser 4 to the contrast space 7.

In other words in a first embodiment shown in figure 1, the means 5 detect the temperature difference between a nebulized fluid 3b and a surrounding space, whereas in a second embodiment shown in figures 2 and 3, the means 5 detect the temperature difference between two portions 7a, 7b of a contrast space 7, that is a first portion 7a that came in contact with the nebulized fluid 3b (i.e. the mark 7a left on the contact surface 7 by the fluid 3b), and a second portion 7b that was not contacted by this fluid 3b.

In a possible embodiment shown in figure 1, the means 5 detect the temperature of the environment downstream of the discharging nozzle 4b after the fluid 3b has been dispensed.

Apposite heating/cooling means 8 (schematically shown in figure 1) operate on the contrast space 7 by cooling or heating it with respect to the fluid 3a. According to a modification not shown, the heating and/or cooling means 8 lead the fluid 3a to a different temperature with respect to the contrast space 7. For example, heating means 8 can heat the fluid 3a before it is discharged from the dispenser 4.

After dispensing the fluid 3b, the means 5 are able to detect the areas downstream of the dispenser 4 in which the nebulized fluid 3b is present. In particular, the means 5 are able to detect areas of the contrast space 7 at a higher temperature, which identify the distribution 2 of the nebulized fluid 3b, whereas the areas at a lower temperature identify the contrast space 7 in which the same fluid 3b did not flow.

Preferably, the means 5 comprise at least one thermographic camera, e.g. two cameras, in order to obtain information about the three-dimensional distribution of the fluid 3b. Typically, the required thermographic cameras must be able to obtain high-resolution images and accurately capture a moment immediately after dispensing the fluid 3b.

Alternatively, the means 5 comprise a plurality of temperature sensors (not shown) conveniently positioned downstream of the dispenser 4.

In a second embodiment, the distribution 2 of the fluid 3b is indirectly detected.

In particular, referring to the embodiments of figures 2 and 3, the contrast space 7 comprises a contact surface 7. Special means 8 heat the surface 7 up to a temperature different from the temperature of the fluid 3a, 3b. Alternatively, means can be provided which lead the temperature of the surface 7 so as to be lower than the temperature of the fluid 3a, 3b.

According to further variations, the means 8 operate on the fluid 3a, 3b by cooling or heating it with respect to the contact surface 7.

Preferably, the contact surface 7 is covered by a film 9 made of a thermal-insulating material, for example Teflon. As better explain hereinafter, the film 9 allows to minimize the time between two subsequent checks of two different dispensers 4. The surface 7 is arranged so as to be incident to the fluid 3b ejected from the dispenser 4. Preferably, the contact surface 7 is arranged so as to be substantially perpendicular to the main emission direction D of the nozzle 4b.

Detecting means 5 allow to detect the mark 7a left by the fluid 3b on the contact surface 7 (or on the film 9, if present).

Similarly to the previous embodiment, these means 5 preferably comprise at least one thermographic camera. Since in this embodiment a two-dimensional image has to be detected, i.e. the mark 7a of the fluid 3b on the contact surface 7 (or the film 9), it is therefore possible to use a single thermographic camera.

Alternatively, a plurality of temperature sensors, suitably distributed on the contact surface 7, can be used.

Once the shape of the mark 7a left by the fluid 3b ejected from the container 4 on the contact surface 7 (or the film 9) has been detected, it is then possible to process the distribution 2 of the fluid 3b exiting from the dispenser 4 by means of known mathematical functions.

Typically, such processing operations are performed by a computer 10.

In order perform this processing, it is also preferable to set, in a known manner, at least the distance between the dispenser 4 and the contact surface 7 (or film 9).

In use, a fluid 3b is discharged from the dispenser 4.

As mentioned, this operation can be controlled so that the dispensing pressure of the fluid 3b can be adjusted, for example by means of a compressor 6a and optionally a valve 6b. Alternatively, for example in the embodiments shown in figures 1 and 3, it is possible to control the operating speed of the actuators controlling the dispensing of the fluid 3a, 3b from the dispenser 6.

The temperature of the fluid 3b is set so as to be different with respect to that of the contrast space 7. As anticipated, this operation can be performed by heating or cooling the fluid 3a, 3b, and/or heating/cooling the contrast space 7.

Subsequently, the distribution of the fluid 3b can be verified by the means 5.

In the previously described first embodiment, the means 5 directly identify (e.g. by a thermographic picture) the distribution of fluid 3b, i.e. the "cone" formed by the fluid 3b downstream of the dispenser 4.

In the second embodiment, the means 5 indirectly identify the distribution of fluid 3b, in particular by detecting a section or mark 7b (stamped on the surface 7 or on the film 9) of the "cone" formed by the fluid 3b downstream of the dispenser 4. Preferably, the means 5 are able to identify the shape of the mark 7a, the size of the mark 7a, the position of the center of the mark 7a.

Having these three data and preferably knowing the position of the discharging nozzle 4b as well, it is possible to define the proper geometry of the distribution 2 of the nebulized fluid 3b and check if the shape, size and direction of the distribution 2 are correct.

As previously mentioned, preferably the surface 7 may be covered by a thermal-insulating film 9, for example made of Teflon. When the fluid 3b collides with the film 9, the latter is very quickly cooled by the fluid itself, but only on the surface. After the detection has been performed by the means 5, the surface 7 heated by the heating means 8 can lead the film 9 back to the initial temperature, so as to allow to quickly perform a subsequent check of another dispenser 4.

Generally, in order to detect the distribution 2 of the fluid 3b, the means 5 are preferably actuated before and after dispensing the fluid 3b. The effect of the fluid 3b on the contrast space 7 can be better highlighted by the difference between the two detections of the means 5. For example, one (or more) thermographic camera is operated to take two pictures: a first picture before dispensing the fluid 3b, and a second picture a preset time after dispensing. The difference between the two pictures highlights the influence of the fluid 3b with respect to the contrast space 7 thereby allowing to obtain information about the distribution 2 of the fluid 3b.

As previously mentioned, preferably a computer 10 processes the data obtained by the means 5 in order to analytically reproduce the shape of the distribution 2 of the fluid 3b.

In particular, by performing the virtual reproduction, typically by means of a computer 10, it will be possible to verify, among other things: the shape, size and space orientation of the distribution 2 of the nebulized fluid 3b ejected from the nozzle 4b of the dispenser 4.

Depending on the result, the just checked dispenser 4 is put on the market or rejected.

## Claims

1. Device (1) for checking the distribution (2) of fluid (3a, 3b) ejected from a nebulizing dispenser (4) of fluid (3a, 3b), of the type comprising a discharging nozzle (4b) of nebulized fluid, said device comprising detecting means (5) for detecting the temperature difference between at least part of said nebulized fluid (3b) ejected from said dispenser (4) and at least one contrast space (7) and/or the temperature difference caused by said nebulized fluid (3b) ejected from said dispenser (4) to said contrast space (7), said contrast space being arranged so as to be at least partially incident to said nebulized fluid (3b), the device being configured so that the temperature of said nebulized fluid (3b) is set so as to be different with respect to that of said contrast space (7).

2. Device according to claim 1, wherein said detecting means (5) are configured to directly or indirectly detect the cone formed by the fluid (3b) downstream of said dispenser (4).

3. Device according to claim 1 or 2, configured to discharge inert gas from said nebulizing dispenser (4).

4. Device (1) according to claim 1, 2 or 3, wherein said contrast space comprises a contact surface (7) having a temperature different from said nebulized fluid (3b) ejected from said dispenser, said contact surface (7) being preferably thermally connected to heating and/or cooling means (8).

5. Device (1) according to claim 1, 2 or 3, wherein said contrast space (7) comprises a volumetric portion of the environment downstream of said discharging nozzle (4b).

6. Device according to one of the preceding claims, wherein said detecting means comprise at least one thermographic sensor to capture at least one image of the temperature difference between at least part of said nebulized fluid ejected from said dispenser and said contrast space and/or the projection of said image onto said contrast space.

7. Device (1) according to one of the preceding claims, wherein said detecting means (5) comprise temperature sensors.

8. Device (1) according to claim 4, wherein said contact surface (7) is at least partially covered by a thermal-insulating material (9), preferably by a film of thermal-insulating material (9).

9. Device according to any of the preceding claims, wherein said device is configured for checking the distribution (2) of fluid (3a, 3b) ejected from a nebulizing dispenser (4) for a cosmetic or a pharmaceutical or a medical fluid.

10. Method for checking the distribution of fluid ejected from a fluid nebulizing dispenser (4) by means of a device (1) according to one of the preceding claims, comprising the steps of:
a) ejecting a nebulized fluid from said discharging nozzle at a temperature different with respect to that of said contrast space (7);
b) detecting the temperature difference between at least part of said nebulized fluid (3b) and said contrast space (7) and/or the temperature variation caused by said nebulized fluid (3b) to said contrast space (7).

11. Method according to claim 10, wherein during said step b), said detection is carried out on a contrast space comprising a contact surface (7) having a temperature different from said nebulized fluid (3b) exiting from said dispenser (4), said contact surface (7) being preferably thermally connected to heating and/or cooling means (8) leading at least part of said contact surface (7) to a temperature different from said nebulized fluid (3b).

12. Method according to claim 10 or 11, wherein said fluid ejected from the nebulizing dispenser (4) is an inert gas.

13. Method according to one of claims 10 to 12 wherein, in said detecting step b), at least one thermographic sensor captures at least one image of the temperature difference between at least part of said fluid (3b) ejected from said dispenser (4) and said contrast space (7).

14. Method according to one of claims 10 to 13 wherein, in said detecting step b), at least one temperature sensor records the temperature difference between said fluid (3b) and said contrast space (7) and/or the temperature variation said fluid (3b) ejected from said dispenser (4) causes to said contrast space (7).

15. Method according to one of claims 10 to 14, wherein in said step a) a control is carried out over the dispensing pressure of said fluid (3b).

## Patentansprüche

1. Vorrichtung (1) zum Überprüfen der Verteilung (2) eines Fluids (3a, 3b), das von einem Zerstäuber (4) für ein Fluid (3a, 3b) ausgestoßen wird, von der Bauart, die eine Ausstoßdüse (4b) für ein zu zerstäubendes Fluid umfasst, wobei die Vorrichtung Nachweismittel (5) zum Nachweisen des Temperaturunterschieds zwischen mindestens einem Teil des von dem Zerstäuber (4) zerstäubten Fluids (3b) und mindestens einem Kontrastraum (7) und/oder des Temperaturunterschieds, der durch das zerstäubte Fluid (3b) verursacht wird, das von dem Zerstäuber (4) in Richtung des Kontrastraums (7) ausgestoßen wird, wobei der Kontrastraum so angeordnet ist, dass er wenigstens teilweise mit dem zerstäubten Fluid (3b) zusammentrifft, wobei die Vorrichtung so konfiguriert ist, dass die Temperatur des zerstäubten Fluids (3b) eingestellt ist, so dass sie verschieden ist von der des Kontrastraumes (7).

2. Vorrichtung gemäß Anspruch 1, wobei die Nachweismittel (5) konfiguriert sind, um direkt oder indirekt den Kegel zu erfassen, der stromabwärts des Zerstäubers (4) durch das Fluid (3b) gebildet wird.

3. Vorrichtung gemäß Anspruch 1 oder 2, die konfiguriert ist, Inertgas von dem Zerstäuber (4) auszustoßen.

4. Vorrichtung (1) gemäß Anspruch 1, 2 oder 3, wobei der Kontrastraum eine Kontaktoberfläche (7) mit einer Temperatur aufweist, die sich von der des zerstäubten Fluids (3b) unterscheidet, das von dem Zerstäuber ausgestoßen wird, wobei die Kontaktoberfläche (7) vorzugsweise mit Heiz- und/oder Kühleinrichtungen (8) thermisch verbunden ist.

5. Vorrichtung (1) gemäß Anspruch 1, 2 oder 3, wobei der Kontrastraum (7) einen volumetrischen Teil der Umgebung stromabwärts der Ausstoßdüse (4b) umfasst.

6. Vorrichtung gemäß einem der vorhergehenden Ansprüche, wobei die Nachweismittel mindestens einen thermographischen Sensor umfassen, um mindestens ein Bild des Temperaturunterschieds zwischen mindestens einem Teil des zerstäubten Fluids, das von dem Zerstäuber ausgestoßen wird, und dem Kontrastraum und/oder der Projektion des Bildes auf dem Kontrastraum zu erfassen.

7. Vorrichtung (1) gemäß einem der vorhergehenden Ansprüche, wobei die Nachweismittel (5) Temperatursensoren umfassen.

8. Vorrichtung (1) gemäß Anspruch 4, wobei die Kontaktoberfläche (7) zumindest teilweise von einem thermisch isolierenden Material (9) bedeckt ist, vorzugsweise von einem Film aus einem thermisch isolierenden Material (9).

9. Vorrichtung gemäß irgendeinem der vorhergehenden Ansprüche, wobei die Vorrichtung konfiguriert ist zur Überprüfung der Verteilung (2) von Fluid (3a, 3b), das von einem Zerstäuber (4) für ein kosmetisches oder ein pharmazeutisches oder ein medizinisches Fluid ausgestoßen wird.

10. Verfahren zum Überprüfen der Verteilung von Fluid, das von einem Fluidzerstäuber (4) ausgestoßen wird, mittels einer Vorrichtung (1) gemäß einem der vorhergehenden Ansprüche enthaltend die Schritte:
a) Ausstoßen eines zerstäubten Fluids aus der Ausstoßdüse bei einer Temperatur, die verschieden ist von der des Kontrastraumes (7);
b) Erfassen des Temperaturunterschieds zwischen mindestens einem Teil des zerstäubten Fluids (3b) und des Kontrastraums (7) und/oder der durch das zerstäubte Fluid (3b) im Kontrastraum (7) verursachten Temperaturschwankung.

11. Verfahren gemäß Anspruch 10, bei dem während des Schritts b) der Nachweis an einem Kontrastraum durchgeführt wird, der eine Kontaktoberfläche (7) aufweist, die eine von der zerstäubten Flüssigkeit (3b), die aus dem Zerstäuber (4) austritt, verschiedene Temperatur hat, wobei die Kontaktoberfläche (7) vorzugsweise mit Heiz- und/oder Kühleinrichtungen (8) thermisch verbunden ist, die bei mindestens einem Teil der Kontaktoberfläche (7) zu einer Temperatur führen, die verschieden von der des zerstäubten Fluids (3b) ist.

12. Verfahren gemäß Anspruch 10 oder 11, wobei das durch den Zerstäuber (4) ausgestoßene Fluid ein Inertgas ist.

13. Verfahren gemäß einem der Ansprüche 10 bis 12, wobei in dem Nachweisschritt b) mindestens ein thermographischer Sensor mindestens ein Bild des Temperaturunterschieds zwischen mindestens einem Teil des aus dem Zerstäuber (4) ausgestoßenen Fluids (3b) und dem Kontrastraum (7) erfasst.

14. Verfahren gemäß einem der Ansprüche 10 bis 13, wobei in dem Nachweisschritt b) mindestens ein Temperatursensor den Temperaturunterschied zwischen dem Fluid (3b) und dem Kontrastraum (7) und/oder die von dem Fluid (3b) in dem Kontrastraum (7) verursachte Temperaturschwankung, das von dem Zerstäuber (4) ausgestoßen wird, aufzeichnet.

15. Verfahren gemäß einem der Ansprüche 10 bis 14, wobei in Schritt a) eine Steuerung über den Abgabedruck des Fluids (3b) durchgeführt wird.

## Revendications

1. Dispositif (1) pour vérifier la distribution (2) de fluide (3a, 3b) éjecté à partir d'un atomiseur (4) de fluide (3a, 3b), du type comprenant une buse d'émission (4b) de fluide nébulisé, ledit dispositif comprenant des moyens de détection (5) pour détecter la différence de température entre au moins une partie dudit fluide nébulisé (3b) éjecté à partir dudit atomiseur (4) et au moins un espace de contraste (7) et/ou la différence de température provoquée par ledit fluide nébulisé (3b) éjecté à partir dudit atomiseur (4) audit espace de contraste (7), ledit espace de contraste étant agencé de façon à être au moins partiellement incident par rapport audit fluide nébulisé (3b), le dispositif étant configuré de sorte que la température dudit fluide nébulisé (3b) est réglée de manière à être différente par rapport à celle dudit espace de contraste (7).

2. Dispositif selon la revendication 1, dans lequel lesdits moyens de détection (5) sont configurés pour détecter directement ou indirectement le cône formé par le fluide (3b) en aval dudit atomiseur (4).

3. Dispositif selon la revendication 1 ou 2, configuré pour émettre un gaz inerte à partir dudit atomiseur (4).

4. Dispositif (1) selon la revendication 1, 2 ou 3, dans lequel ledit espace de contraste comprend une surface de contact (7) ayant une température différente dudit fluide nébulisé (3b) éjecté à partir dudit atomiseur, ladite surface de contact (7) étant de préférence connectée thermiquement à des moyens de chauffage et/ou de refroidissement (8).

5. Dispositif (1) selon la revendication 1, 2 ou 3, dans lequel ledit espace de contraste (7) comprend une partie volumétrique de l'environnement en aval de ladite buse d'émission (4b).

6. Dispositif selon l'une des revendications précédentes, dans lequel lesdits moyens de détection comprennent au moins un capteur thermographique pour saisir au moins une image de la différence de température entre au moins une partie dudit fluide nébulisé éjecté à partir dudit atomiseur et ledit espace de contraste et/ou la projection de ladite image sur ledit espace de contraste.

7. Dispositif (1) selon l'une des revendications précédentes, dans lequel lesdits moyens de détection (5) comprennent des capteurs de température.

8. Dispositif (1) selon la revendication 4, dans lequel ladite surface de contact (7) est au moins partiellement recouverte par un isolant thermique (9), de préférence par un film d'isolant thermique (9).

9. Dispositif selon l'une quelconque des revendications précédentes, dans lequel ledit dispositif est configuré pour vérifier la distribution (2) de fluide (3a, 3b) éjecté à partir d'un atomiseur (4) pour un fluide cosmétique, pharmaceutique ou médical.

10. Procédé pour vérifier la distribution de fluide éjecté à partir d'un atomiseur de fluide (4) au moyen d'un dispositif (1) selon l'une des revendications précédentes, comprenant les étapes de :
a) éjection d'un fluide nébulisé à partir de ladite buse d'émission à une température différente par rapport à celle dudit espace de contraste (7) ;
b) détection de la différence de température entre au moins une partie dudit fluide nébulisé (3b) et ledit espace de contraste (7) et/ou de la variation de température provoquée par ledit fluide nébulisé (3b) audit espace de contraste (7).

11. Procédé selon la revendication 10, dans lequel pendant ladite étape b), ladite détection est réalisée sur un espace de contraste comprenant une surface de contact (7) ayant une température différente dudit fluide nébulisé (3b) sortant dudit atomiseur (4), ladite surface de contact (7) étant de préférence connectée thermiquement à des moyens de chauffage et/ou de refroidissement (8) portant au moins une partie de ladite surface de contact (7) à une température différente dudit fluide nébulisé (3b).

12. Procédé selon la revendication 10 ou 11, dans lequel ledit fluide éjecté à partir de l'atomiseur (4) est un gaz inerte.

13. Procédé selon l'une des revendications 10 à 12 dans lequel, dans ladite étape de détection b), au moins un capteur thermographique saisit au moins une image de la différence de température entre au moins une partie dudit fluide (3b) éjecté à partir dudit atomiseur (4) et ledit espace de contraste (7).

14. Procédé selon l'une des revendications 10 à 13 dans lequel, dans ladite étape de détection b), au moins un capteur de température enregistre la différence de température entre ledit fluide (3b) et ledit espace de contraste (7) et/ou la variation de température que ledit fluide (3b) éjecté à partir dudit atomiseur (4) provoque audit espace de contraste (7).

15. Procédé selon l'une des revendications 10 à 14, dans lequel dans l'étape a) un contrôle est réalisé sur la pression d'atomisation dudit fluide (3b).
